# EUROPÄISCHE PATENTSCHRIFT

(11) **EP 2 429 484 B1**
(45) Veröffentlichungstag und Bekanntmachung des Hinweises auf die Patenterteilung: **25.03.2015**
(21) Anmeldenummer: 10713685.5
(22) Anmeldetag: 16.04.2010
(51) Int. Cl.: A61K 8/49, A61Q 5/02

(54) **HAARPFLEGE MIT ACETYLPYRIDINIUMSALZEN**
HAIR CARE AGENT CONTAINING ACETYLPYRIDINIUM SALTS
SOIN CAPILLAIRE CONTENANT DES SELS D'ACÉTYLPYRIDINIUM

(30) Priorität: 15.05.2009 DE 102009003154
(43) Veröffentlichungstag der Anmeldung: 21.03.2012
(73) Patentinhaber: Henkel AG & Co. KGaA, 40589 Düsseldorf (DE)
(72) Erfinder: GROß, Wibke, 41836 Hückelhoven (DE); KNÜBEL, Georg, 40219 Düsseldorf (DE); MUCHA, Thomas, 40699 Erkrath (DE); SCHULZE ZUR WIESCHE, Erik, 20144 Hamburg (DE)
(86) Internationale Anmeldenummer: PCT/EP2010/055033
(87) Internationale Veröffentlichungsnummer: WO 2010/130526

(56) Entgegenhaltungen:
- WO-A1-2005/097047
- WO-A1-2009/135700
- WO-A1-2010/022996
- WO-A2-2010/029006
- DE-A1-102007 047 685
- US-B1- 6 371 993

## Beschreibung

Die vorliegende Erfindung betrifft die Verwendung wasserstoffperoxidfreier Haarbehandlungsmittel, basierend auf einer speziellen Kombination aus einem Acetylpyridiniumsalz mit einem anionischen, zwitterionischem und/oder einem amphoteren Tensid, zur Steigerung der haarkonditionierenden Eigenschaften.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten.

Nicht zuletzt durch die starke Beanspruchung der Haare, beispielsweise durch das Färben oder Dauerwellen, durch die häufige Reinigung der Haare mit Shampoos und anschließendes Föhnen mit Föhnhitze, durch mechanische Belastungen wie Kämmen und Frisieren oder durch Umweltbelastungen, wie Sonnenlicht, Meerwasser oder Chlorwasser, nimmt die Bedeutung von Pflegeprodukten mit möglichst lang anhaltender Wirkung zu. Wenn der Konsument durch Reduktion von haarkosmetischen Behandlungen den Schädigungsgrad seiner Haare gering halten kann, so ist es doch nicht möglich, sich Umwelteinflüssen komplett zu entziehen. Dies führt dazu, dass die Haare durch die Beanspruchung sowohl äußerlich als auch in ihrer Struktur geschädigt werden können, was ihnen ein unattraktives Ansehen verleiht, das sich durch mangelnde Glätte, Weichheit, mangelnden Glanz, aber auch durch eine schlechtere Kämmbarkeit, Haarbruch oder Spliss bemerkbar machen kann.

Daher ist es seit langem üblich, die Haare einer speziellen Nachbehandlung zu unterziehen, um den Haaren und der Kopfhaut Pflegestoffe zuzuführen, die den Haaren wieder ein schönes äußeres Aussehen verleihen und die Haarstruktur kräftigen sowie die Kopfhaut pflegen bzw. vor dem Austrocknen schützen. Bei solchen Nachbehandlungen werden, üblicherweise in Form einer Spülung, die Haare mit speziellen Wirkstoffen, beispielsweise quaternären Ammoniumsalzen oder speziellen Polymeren, behandelt. Durch diese Behandlung können, je nach Formulierung, die Kämmbarkeit, der Halt, die Fülle sowie der Glanz der Haare verbessert, und die Splissrate verringert werden.

Es wurde allerdings festgestellt, dass die Haare durch Färben, Dauerwellen oder Blondieren und anschließendes Waschen und Konditionieren fühlbar an Volumen und Haarfülle verlieren können, so dass die Reinigung- und Konditionierung (die nach einem Färbe-, Bleich- oder Dauerwellvorgang unbedingt erforderlich ist) auch erhebliche Nachteile für die Ästhetik der Haare mit sich bringt.

Trotz der mannigfaltigen Bemühungen der Haarpflege- und -regeneration in den letzten Jahrzehnten stellen sich immer neue Problemstellungen bei der Haarbehandlung, für die die Fachwelt fortwährend an neuen Lösungen arbeiten muss. Die Suche nach neuen Inhaltsstoffen mit positivem Effekt auf die Haarstruktur ist demzufolge nach wie vor hochaktuell. Es ist die Aufgabe dieser Erfindungsmeldung, ein neues Mittel zum Pflegen von Haaren bereitzustellen, welches einen strukturierenden Effekt auf die Haarfaser ausübt und es hierdurch ermöglicht, bereits im Haar vorhandene Schäden im Sinne eines "Repair-Effektes" rückgängig zu machen.

Aufgabe der vorliegenden Erfindung ist daher die Bereitstellung reinigender und/oder konditionierender Haarbehandlungsmittel, die das Haarvolumen und die Haarfülle insbesondere von vorher gefärbten, gebleichten und/oder verformten Haaren nicht negativ beeinflussen. Es ist insbesondere Aufgabe der vorliegenden Anmeldung, Mittel zur Regeneration von Umwelteinflüssen, UV-Lichteinstrahlurig, Hitze oder speziellen Haarbehandlungen bereitzustellen.

Ein besonderer Fokus liegt insbesondere darin, ein Mittel bereitzustellen, welches nicht lediglich durch Einwirkung auf die äußere Schicht des Haares (Cuticula) eine Pflegewirkung entfaltet, sondern welches in die Haarfaser hinein zu diffundieren vermag und auf diese Weise eine Stärkung der Haarfaser über den gesamten Querschnitt des Cortex bewirkt.

Zur Quantifizierung von Haarschädigungen bzw. der Stärkung der Haarfaser nach einer Haarbehandlung werden häufig die Zug-Dehnungs-Eigenschaften einzelner Haarfasern bestimmt. Die dabei anfallenden Kraft-Dehnungskurven durchschreiten drei unterschiedliche Bereiche, wobei sich aus Zug-Dehnungs-Messungen im Hook'schen Bereich (bis 5 % Dehnung) das Elastizitätsmodul (E-Modul, Young's modulus) als charakteristische Messgröße bestimmen lässt.

Oberflächliche Schädigung oder Abräsionsschädigungen, die vor allem die Kutikula betreffen, verändern die Zug-Dehnungs-Werte nur geringfügig. Die in handelsüblichen Kuren und Spülungen vorhandenen Aktivsubstanzen sind hauptsächlich kationische Tenside bzw. Polymere. Diese kationischen Verbindungen lagern sich durch elektrostatische Wechselwirkungen mit den anionischen Gruppen des Haares außen an die Cuticula an. Eine Strukturverbesserung im Inneren der Haarfaser lässt sich mit diesen Pflegeprodukten daher nicht erreichen.

Schädigungen mit negativen Auswirkungen auf die Struktur des Cortex und die Festigkeit der Haare resultieren in reduzierten Werten des E-Moduls, Substanzen, die die Festigkeit der Haarstruktur verbessern, führen zu einer Erhöhung des E-Moduls. Die Bestimmung von Zug-Dehnungs-Werten und insbesondere des Elastizitäts-Moduls ist demnach eine optimale Messmethode zur Identifizierung von Substanzen mit haarstrukturierendem Effekt.

Vollkommen überraschend konnte nun gefunden werden, dass es möglich ist, die vorgenannten Aufgaben zu lösen, indem Haarreinigungs- und/oder -konditioniermittel zur Verfügung gestellt werden, die neben einem anionischen, zwitterionischem und/oder amphoteren Tensid mindestens ein 4-Acetylpyridiniumderivat vom Typ eines 4-Acetylpyridiniumsalzes enthalten. WO2005/097047A1 offenbart 1-Methylpyridinium-3-carboxylat (Trigonellin) und dessen Salze als Repair-Wirkstoff für geschädigtes Haar.

Die Substanzklasse der erfindungsgemäßen 4-Acetylpyridiniumderivate ist aus der Literatur bereits als Mittel zur Erzeugung von Färbungen auf Haaren (DE 19745356) oder aus Mitteln zur Aufhellung von Haaren (DE102007047685) bekannt. In diesem Zusammenhang nicht beschrieben sind die positiven Effekte der erfindungsgemäßen Substanzen auf die Haarqualität in wasserstoffperoxidfreien Mitteln. Es war daher nicht vorhersehbar, dass die erfindungsgemäße Aufgabe durch Einsatz einer Kombination aus 4-Acetylpyridiniumderivaten und bestimmten Tensidsystemen gelöst werden kann.

Ein erster Gegenstand der Erfindung ist daher die Verwendung eines kosmetischen Haarbehandlungsmittels, das frei ist von Wasserstoffperoxid und/oder dessen Anlagerungsverbindungen an organische und anorganische Verbindungen ist, enthaltend in einem kosmetischen Träger
a) mindestens ein oberflächenaktives Mittel, ausgewählt aus der Gruppe der anionischen, amphoteren und/oder zwitterionischen Tenside, und
b) mindestens ein Acetylpyridiniumderivat der Formel (I), 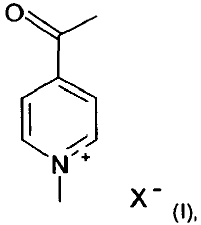 worin
   - R1: für eine C₁-C₆-Alkylgruppe, eine C₂-C₆-Alkenylgruppe, eine C₂-C₆-Hydroxyalkylgruppe, eine C₁-C₆-Alkoxy-C₂-C₆-alkylgruppe, eine Carboxy-C₁-C₆-alkylgruppe, eine Aryl-C₁-C₆-alkylgruppe, eine Heteroaryl-C₁-C₆-alkylgruppe, eine Arylgruppe oder eine Heteroarylgruppe und
   - X⁻: für ein physiologisch verträgliches Anion stehen,
zur Reduktion von Haarschädigungen im Inneren der Haare und zur Steigerung der Elastizität der Haare.

Es hat sich gezeigt, dass solche verwendeten Mittel in der Lage sind, insbesondere das Haar in seinem Faserinneren zu stärken und Strukturschädigungen zu verringern. Insgesamt wird bei Anwendung des Mittels ein "Repair-Effekt" beobachtet.

Die Mittel enthalten die Wirkstoffe in einem kosmetischen Träger. Dieser kosmetische Träger ist bevorzugt wässrig, alkoholisch oder wässrig-alkoholisch. Solche Träger sind beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Ein wässriger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel.

Bevorzugt verwendete Mittel sind dadurch gekennzeichnet, dass sie zusätzlich ein nichtwässriges Lösungsmittel enthalten, wobei besonders bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer Konzentration von 2 bis 10 Gew.-%, jeweils bezogen auf das Mittel, enthalten.

Das erfindungsgemäß verwendete Mittel ist dadurch gekennzeichnet, dass es frei von Wasserstoffperoxid und/oder dessen Anlagerungsverbindungen an organische und anorganische Verbindungen ist. Es ist daher insbesondere nicht als Aufhell- oder Bleichmittel zu verstehen. Unter Mitteln, die frei von Wasserstoffperoxid und/oder dessen Anlagerungsverbindungen an organische und anorganische Verbindungen sind, sind solche Mittel zu verstehen, die Wasserstoffperoxid und/oder dessen Anlagerungsverbindungen an organische und anorganische Verbindungen in einer Gesamtmenge von weniger als 0,5 Gew.-%, insbesondere weniger als 0,1 Gew.-% und ganz besonders weniger als 0,01 Gew.-% enthalten. Wasserstoffperoxid und/oder dessen Anlagerungsverbindungen an organische und anorganische Verbindungen sind im Sinne der vorliegenden Erfindung Wasserstoffperoxid selbst oder feste Anlagerungsverbindungen von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidinonn H₂O₂ (n ist eine positive ganze Zahl größer 0), Harnstoffperoxid und Melaminperoxid, aber auch Peroxosalze, wie Ammoniumperoxodisulfat, Alkalimetallperoxodisulfate, Ammoniumperoxomonosulfat, Alkalimetallperoxomonosulfate, Alkalimetallperoxodiphosphate und Erdalkalimetallperoxide.

Als ersten wesentlichen Inhaltsstoff enthalten die erfindungsgemäß verwendeten Mittel ein Acetylpyridiniumderivat gemäß Formel (I). Im Folgenden werden Beispiele für die als Substituenten der Verbindungen der Formel (I) genannten Reste aufgezählt:
Beispiele für C₁-C₆-Alkylreste sind die Gruppen -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃.

Beispiele für eine C₂-C₆-Alkenylgruppe sind eine Prop-2-enylgruppe (Allylgruppe), eine 2-Methyl-prop-2-enylgruppe, eine But-3-enylgruppe, eine But-2-enylgruppe, eine Pent-4-enylgruppe oder eine Pent-3-enylgruppe, wobei die Prop-2-enylgruppe bevorzugt ist.

Beispiele für eine C₂-C₆-Hydroxyalkylgruppe sind -CH₂CH₂OH, -CH₂CH₂CH₂OH, -CH₂CH(OH)CH₃ und -CH₂CH₂CH₂CH₂OH, wobei die Gruppe -CH₂CH₂OH bevorzugt ist.

Beispiele für C₁-C₆-Alkoxy-C₂-C₆-alkylgruppen sind die Gruppen -CH₂CH₂OCH₃, -CH₂CH₂CH₂OCH₃, -CH₂CH₂OCH₂CH₃, -CH₂CH₂CH₂OCH₂CH₃, -CH₂CH₂OCH(CH₃)₂, -CH₂CH₂CH₂OCH(CH₃)₂.

Beispiele für eine Carboxy-C₁-C₆-alkylgruppe sind die Carboxymethylgruppe, die 2-Carboxy-ethylgruppe oder die 3-Carboxypropylgruppe.

Beispiele für Aryl-C₁-C₆-alkylgruppen sind die Benzylgruppe und die 2-Phenylethylgruppe. Beispiele für eine Heteroaryl-C₁-C₆-alkylgruppe sind die Pyridin-2-ylmethylgruppe, die Pyridin-3-yl-methylgruppe, die Pyridin-4-ylmethylgruppe, die Pyrimidin-2-ylmethylgruppe, die Pyrrol-1-ylmethyl-gruppe, die Pyrrol-1-ylethylgruppe, die Pyrazol-1-ylmethylgruppe oder die Pyrazol-1-ylethylgruppe. Beispiele für eine Arylgruppe sind die Phenylgruppe, die 1-Naphthylgruppe oder die 2-Naphthylgruppe.

Beispiele für eine Heteroarylgruppe sind die Pyridin-2-ylgruppe, die Pyridin-3-ylgruppe, die Pyridin-4-ylgruppe, die Pyrimidin-2-ylgruppe, die Pyrrol-1-ylgruppe, die Pyrrol-2-ylgruppe, die Pyrazol-1-ylgruppe, die Pyrazol-3-ylgruppe oder die Pyrazol-2-ylgruppe.

In einer Ausführungsform der vorliegenden Erfinung sind solche Verbindungen gemäß Formel (I) bevorzugt, wenn der Rest R der allgemeinen Struktur (I) für eine C₁-C₆-Alkylgruppe, für eine C₂-C₆-Alkenylgruppe oder für eine C₂-C₆-Hydroxyalkylgruppe steht.

Es ist erfindungsgemäß besonders bevorzugt, wenn der Rest R für eine C₁-C₆-Alkylgruppe, bevorzugt für Methyl, Ethyl, n-Propyl oder Isopropyl und insbesondere bevorzugt für Methyl, steht.

Es ist bevorzugt, wenn das Anion X⁻ gemäß Formel (I) ausgewählt wird aus Halogenid, insbesondere Chlorid, Bromid und Iodid, Benzolsulfonat, p-Toluolsulfonat, C₁-C₄-Alkylsulfonat, Trifluormethansulfonat, Acetat, Triflluoracetat, Perchlorat, ½ Sulfat, Hydrogensulfat, Tetrafluorborat, Hexafluorphosphat oder Tetrachlorzinkat. Erfindungsgemäß besonders begünstigt ist es, wenn das physiologisch verträgliche Anion X⁻ für ein Halogenidion (insbesondere Chlorid oder Bromid), Hydrogensulfat, ½ Sulfat, p-Toluolsulfonat, Benzolsulfonat oder Acetat steht.

Geeignete Acetylpyridiniumderivate sind dabei die physiologisch verträglichen Salze, die als Kation ein Acetylpyridiniumderivat, ausgewählt aus 4-Acetyl-1-methylpyridinium, 4-Acetyl-l-allylpyridinium und 4-Acetyl-1-(2-hydroxyethyl)pyridinium, enthalten.

Insbesondere sind solche Mittel erfindungsgemäß geeignet, die dadurch gekennzeichnet sind, dass das Acetylpyridiniumderivat gemäß Formel (I) ausgewählt ist aus der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumbromid, 4-Acetyl-1-methylpyridiniumchlorid, 4-Acetyl-1-methyl-pyridiniumhydrogensulfat, 4-Acetyl-1-methylpyridiniumacetat, 4-Acetyl-1-allylpyridinium-p-toluolsulfonat, 4-Acetyl-1-allylpyridinium-benzolsulfonat, 4-Acetyl-1-allylpyridiniumbromid, 4-Acetyl-1-allylpyridiniumchlorid, 4-Acetyl-1-allylpyridiniumhydrogensulfat und/oder 4-Acetyl-1-allyl-pyridiniumacetat.

Dabei sind besonders vorteilhafte Mittel dadurch gekennzeichnet, dass das Acetylpyridiniumderivat gemäß Formel (I) ausgewählt ist aus 4-Acetyl-1-methylpyridinium-p-toluolsulfonat.

In einer Ausführungsform enthalten die erfindungsgemäß verwendeten Haarbehandlungsmittel die Acetylpyridiniumderivate der Formel (I) zu 0,05 bis 10 Gew.-%, bevorzugt zu 0,1 bis 7,5 Gew.-%, bevorzugt zu 0,2 bis 6,5 Gew.-% und insbesondere zu 0,5 bis 5 Gew.-%, jeweils bezogen auf das Gesamtgewicht des anwendungsbereiten Haarbehandlungsmittels.

Als weiteren wesentlichen Inhaltsstoff enthalten die erfindungsgemäß verwendeten Mittel mindestens ein oberflächenaktives Mittel, ausgewählt aus der Gruppe der anionischen, amphoteren und/oder zwitterionischen Tenside.

Als anionische Tenside eignen sich in erfindungsgemäß verwendeten Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sultat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel RO(CH₂CH₂O)ₓCH₂COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare α-Olefinsulfonate mit 8 bis 24 C-Atomen,
- Sulfonate ungesättigter Fettsäuren mit 8 bis 24 C-Atomen und 1 bis 6 Doppelbindungen,
- α-Sulfofettsäuremethylester von Fettsäuren mit 8 bis 30 C-Atomen,
- Alkylsulfate und Alkylethersulfate der Formel RO(CH₂CH₂O)ₓSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- Ethercarbonsäuren von Alkylpolyglycosiden der Formel R-(Gl_{y})ₓCH₂COOH, in der R für eine Alkylgruppe mit 8 bis 30 C-Atomen, Gly für ein Glycosid, ausgewählt aus Pyranosiden, und x für eine Zahl von 1 bis 5 stehen,
- sulfatierte Hydroxyalkylpolyethylen- und/oder Hydroxyalkylenpropylenglykolether,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel 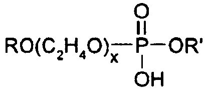 in der R bevorzugt für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht,
- sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht,
- Monoglyceridsulfate und Monoglyceridethersulfate der Formel (MGS) 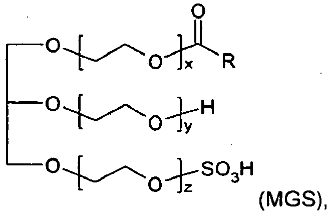 in der R für einen linearen oder verzweigten Alkylrest mit 6 bis 22 Kohlenstoffatomen, und x, y und z in Summe für 0 oder für Zahlen von 1 bis 30, vorzugsweise 2 bis 10 stehen.

Bevorzugte anionische Tenside für alle erfindungsgemäß verwendeten Haarbehandlungsmittel sind lineare und verzweigte Fettsäuren mit 8 bis 30 C-Atomen,
geradkettige und verzweigte Alkylsulfate und Alkylpolyglykolethersulfate, Ethercarbonsäuren der Formel RO(CH₂CH₂O)ₓCH₂COOH, in der R eine lineare Alkylgruppe mit 10 bis 18 C-Atomen und x = 0 oder 1 bis 12 ist sowie
Ethercarbonsäuren von Alkylpolyglycosiden der Formel R-(Gl_{y})ₓCH₂COOH, in der R für eine Alkylgruppe mit 10 bis 18 C-Atomen, Gly für ein Glycosid, ausgewählt aus Pyranosiden, und x für eine Zahl von 1 bis 5 stehen.

Weiterhin besonders bevorzugt verwendet sind Mittel, die als anionisches Tensid mindestens eine lineare und verzweigte, gegebenenfalls ungesättigte Fettsäure mit 10 bis 22 C-Atomen, und/oder eine Ethercarbonsäure von Alkylpolyglycosiden der Formel R-(Gl_{y})ₓCH₂COOH, in der R für eine Alkylgruppe mit 10 bis 18 C-Atomen, Gly für ein Glycosid, ausgewählt aus Pyranosiden, und x für eine Zahl von 1 bis 5 stehen, enthalten.

Als Fettsäuren können eingesetzt werden lineare und/oder verzweigte, gesättigte und/oder ungesättigte Fettsäuren mit 6 bis 30 Kohlenstoffatomen. Bevorzugt sind Fettsäuren mit 10 bis 22 Kohlenstoffatomen. Typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Isopalmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist der Einsatz von Stearinsäure.

Eine bevorzugte Ausführungsform der der vorliegenden Erfindung ist dadurch gekennzeichnet, dass das erfindungsgemäß verwendete Haarbehandlungsmittel als oberflächenaktives Mittel mindestens ein anionisches Tensid enthält.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das C₁₂-C₁₈-Acylsarcosin.

Dabei handelt es sich üblicherweise um Haarbehandlungsmittel, wie beispielsweise Shampoos, die in einer bevorzugten Ausführungsform mindestens ein anionisches Tensid und mindestens ein amphoteres und/oder zwitterionisches Tensid in einem Verhältnis von anionischem(n) Tensid(en) zu amphoterem(n) bzw. zwitterionischem(n) Tensid(en) von 3:1 bis 1:2 bevorzugt von 2:1 bis 1:1, enthalten.

Als Tensidkombination mit der effektivsten Kopfhaut-Schonung hat sich für die erfindungsgemäß verwendeten Haarbehandlungsmittel, die als Shampoo konfektioniert werden, eine Kombination aus Alkylethersulfaten und Dialkalimetall Cocoamphodiacetaten in einem Verhältnis von 3:1 bis 1:2, bevorzugt von 2:1 bis 1:1 herausgestellt.

Die Erfindung betrifft bevorzugt die Verwendung solcher Mittel, die dadurch gekennzeichnet sind, dass die oberflächenaktiven Mittel, ausgewählt aus der Gruppe der anionischen, amphoteren und/oder zwitterionischen Tenside, in einer Gesamtmenge von 0,1 bis 50 Gew.-%, bevorzugt 0,5 bis 25 Gew.-%, mehr bevorzugt 1 bis 20 Gew.-% und insbesondere 2 bis 15 Gew.-%, jeweils bezogen auf das Gesamtgewicht des anwendungsbereiten Haarbehandlungsmittels, enthalten sind.

Ziel der vorliegenden Erfindung ist es weiterhin, die Haare und die Kopfhaut mit Haut- und Haarpflegestoffen nachhaltig zu versorgen.

Bei den vorgenannten Wirkstoffen handelt es sich um mindestens einen Stoff aus der Gruppe der Proteinhydrolysate, der Vitamine, der Aminosäuren, der Pflanzenextrakte und/oder Ectoin, die in den erfindungsgemäß verwendeten Haarbehandlungsmitteln, bezogen auf deren Gesamtgewicht, in einer Menge von 0,001 bis 10 Gew.-%, bevorzugt in einer Menge von 0,01 bis 7,5 Gew.-% und insbesondere in einer Menge von 0,1 bis 5 Gew.-% eingesetzt werden.

Eine weitere Ausführungsform der vorliegenden Erfindung ist daher dadurch gekennzeichnet, dass das erfindungsgemäß verwendete Haarbehandlungsmittel weiterhin mindestens einen Pflegestoff aus der Gruppe der Proteinhydrolysate und/oder der Vitamine und/oder der Aminosäuren und/oder der Pflanzenextrakte und/oder Ectoin enthält.

Es kann erfindungsgemäß bevorzugt sein, dass mehrere Stoffe aus der vorgenannten Gruppe der Haut- und Haarpflegewirkstoffe enthalten sind. Erfindungsgemäß ist eine Kombination aus mindestens einem Vitamin und mindestens einem Pflanzenextrakt, einer Aminoäure oder Ectoin oder eine Kombination aus mindestens einem Proteinhydrolysat und mindestens einem Pflanzenextrakt, einer Aminosäure oder Ectoin. Besonders bevorzugt ist die Kombination aus Vitaminen und Aminosäuren, Vitaminen und Ectoin sowie Proteinhydrolysaten und Aminosäuren.

Unter erfindungsgemäß bevorzugten Vitaminen, Provitaminen und Vitaminvorstufen sowie deren Derivaten sind solche Vertreter zu verstehen, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,01 bis 1 Gew.-%, bezogen auf die gesamte Zubereitung.

Zur Vitamin B-Gruppe oder zu dem Vitamin B-Komplex gehören u. a.
- Vitamin B₁ (Thiamin);
- Vitamin B₂ (Riboflavin);
- Vitamin B₃, wobei unter dieser Bezeichnung häufig die Verbindungen Nicotinsäure und Nicotinsäureamid (Niacinamid) geführt werden. Erfindungsgemäß bevorzugt ist das Nicotinsäureamid, das in den erfindungsgemäßen Mitteln bevorzugt in Mengen von 0,05 bis 1 Gew.-%, bezogen auf das gesamte Mittel, enthalten ist.
- Vitamin B₅ (Pantothensäure und Panthenol). Im Rahmen dieser Gruppe wird bevorzugt das Panthenol, insbesondere D-Panthenol, eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. -Vitamin B₆ (Pyridoxin sowie Pyridoxamin und Pyridoxal).
Die genannten Verbindungen der Vitamin B-Gruppe sind in den erfindungsgemäß verwendeten Haarbehandlungsmitteln bevorzugt in Mengen von 0,01 bis 2 Gew.-%, besonders bevorzugt von 0,03 bis 1 Gew.-%, bezogen auf das gesamte Haarbehandlungsmittel, enthalten.

Vitamin C (Ascorbinsäure). Vitamin C wird in den erfindungsgemäß verwendeten Haarbehandlungsmitteln bevorzugt in Mengen von 0,01 bis 3 Gew.-%, bezogen auf das gesamte Haarbehandlungsmittel, eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,01 bis 1 Gew.-%, bezogen auf das gesamte Haarbehandlungsmittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäß verwendeten Haarbehandlungsmitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäß verwendeten Haarbehandlungsmittel Vitamine, Provitamine und Vitaminvorstufen aus den Gruppen A, B, E und H. Selbstverständlich können auch mehrere Vitamine und Vitaminvorstufen gleichzeitig enthalten sein. In einer besonders bevorzugten Ausführungsform der Erfindung enthalten die Haarbehandlungsmittel zur Unterstützung des Feuchthaltevermögens der Kopfhaut und/oder der Haare D-Panthenol, fakultativ in der Mischung mit einem der anderen vorgenannten Vitaminkomponenten.

Erfindungsgemäß geeignete Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen, marinen oder synthetischen Ursprungs in einer Menge von 0,01 bis 10 Gew.-%, bevorzugt von 0,05 bis 5 Gew.-% und insbesondere von 0,1 bis 3 Gew.-%, bezogen auf das Gesamtgewicht des erfindungsgemäß verwendeten Haarbehandlungsmittels, eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Kerasol^{®} (Croda) oder ProSina^{®} (Croda) vertrieben.

Erfindungsgemäß geeignete Proteinhydrolysate pflanzlichen Ursprungs sind z. B. Soja-, Mandel-, Erbsen-, Kartoffel-, Reis- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda) oder Crotein^{®} (Croda) vertrieben.

Als besonders geeignet hat sich der Zusatz eines Keratin-, Kollagen- oder Seidenproteinhydrolysats zu der erfindungsgemäßen Wirkstoffkombination herausgestellt. Insbesondere bevorzugt in Bezug auf eine Erhöhung des Feuchthaltevermögens ist das Keratinhydrolysat.

Als weiteren bevorzugten Haut- und/oder Haarpflegewirkstoff können die erfindungsgemäß verwendeten Haarbehandlungsmittel mit besonderem Vorzug eine oder mehrere Aminosäuren enthalten. Aminosäuren wirken ebenfalls hautbefeuchtend und stabilisieren aufgrund ihrer Pufferwirkung den Säuremantel der Haut, d.h. sie dienen dem (Kopf)hautschutz. Erfindungsgemäß geeignete Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3',4'-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäß verwendete Haarbehandlungsmittel enthalten eine oder mehrere Aminosäuren in engeren Mengenbereichen. Hier sind erfindungsgemäß bevorzugt verwendete Haarbehandlungsmittel dadurch gekennzeichnet, dass sie als Pflegestoff, bezogen auf ihr Gewicht, 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), vorzugsweise aus der Gruppe Glycin und/oder Alanin und/oder Ariginin und/oder Serin und/oder Valin und/oder Lysin und/oder Leucin und/oder Threonin, enthalten. Insbesondere bevorzugt für den Einsatz in den erfindungsgemäß verwendeten Haarbehandlungsmitteln sind die Aminosäuren Glycin, Alanin und/oder Arginin.

Als weiteren bevorzugten Haut- und/oder Haarpflegewirkstoff können die erfindungsgemäß verwendeten Haarbehandlungsmittel mit besonderem Vorzug zur mechanischen und sensorischen Stärkung der Haare Pflanzenextrakte enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Eichenrinde, Echinacea, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Moringa, Ginseng und Ingwerwurzel bevorzugt.

Erfindungsgemäß ganz besonders bevorzugt aufgrund ihrer haarkräftigenden Wirkung sowie ihrer unterstützenden Wirkung in Bezug auf die Haarfülle sind die Extrakte aus Grünem Tee, Kamille, Mandel, Aloe Vera und Moringa. Insbesondere bevorzugt ist erfindungsgemäß ein Moringa-Extrakt, beispielsweise ein unter dem Handelsnamen Puricare^{®} LS 9658 oder Moringa Oleifera^{®} vertriebenes Produkt.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 0,2 bis 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäß verwendeten Haarbehandlungsmitteln Mischungen aus mehreren, insbesondere aus zwei, verschiedenen Pflanzenextrakten einzusetzen. Erfindungsgemäß besonders bevorzugt verwendete Haarbehandlungsmittel sind dadurch gekennzeichnet, dass sie, bezogen auf ihr Gewicht, 0,001 bis 10 Gew.-%, bevorzugt 0,005 bis 5 und insbesondere 0,01 bis 2 Gew.-%, Pflanzenextrakte (entsprechend dem Aktivsubstanzgehalt des Extrakts) enthalten.

Als weiteren Haut- und/oder Haarpflegewirkstoff können die erfindungsgemäß verwendeten Haarbehandlungsmittel ebenfalls bevorzugt Ectoin ((4S)-2-Methyl-1,4,5,6-Tetrahydropyrimidin-4-Carbonsäure) enthalten. Erfindungsgemäß bevorzugt verwendete Haarbehandlungsmittel sind dadurch gekennzeichnet, dass sie, bezogen auf ihr Gewicht, 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% (S)-2-Methyl-1,4,5,6-tetrahydro-4-pyrimidincarbonsäure (Ectoin) sowie die physiologisch verträglichen Salze dieser Verbindung und/oder (S,S)-5-Hydroxy-2-methyl-1,4,5,6-tetrahydro-4-pyrimidin-carbonsäure (Hydroxyectoin) sowie die physiologisch verträglichen Salze dieser Verbindung, enthalten.

Eine weitere Ausführungsform der vorliegenden Erfindung ist dadurch gekennzeichnet, dass das Mittel als zusätzliche Pflegekomponente D-Panthenol, Keratinhydrolysat, Glycin, Serin, Arginin, Moringa Oleifera-Extrakt und/oder Ectoin, enthält.

In einer weiteren Ausführungsform der Einfindung enthalten die erfindungsgemäß verwendeten Haarbehandlungsmittel, bezogen auf ihr Gewicht, neben der erfindungsgemäßen Wirkstoffkombination weiterhin eine Mischung aus 0,01 bis 2 Gew.-%, bevorzugt 0,05 bis 1,5 Gew.-% und insbesondere 0,1 bis 1 Gew.-% D-Panthenol und 0,01 bis 2 Gew.-%, bevorzugt 0,05 bis 1,5 Gew.-% und insbesondere 0,1 bis 1 Gew.-% Glycin, Serin und/oder Arginin.

In einer weiteren Ausführungsform der Einfindung enthalten die erfindungsgemäß verwendeten Haarbehandlungsmittel, bezogen auf ihr Gewicht, neben der erfindungsgemäßen Wirkstoffkombination weiterhin eine Mischung aus 0,01 bis 2 Gew.-%, bevorzugt 0,05 bis 1,5 Gew.-% und insbesondere 0,1 bis 1 Gew.-% D-Panthenol und 0,01 bis 2 Gew.-%, bevorzugt 0,05 bis 1,5 Gew.-% und insbesondere 0,1 bis 1 Gew.-% Ectoin.

In einer weiteren Ausführungsform der Einfindung enthalten die erfindungsgemäß verwendeten Haarbehandlungsmittel, bezogen auf ihr Gewicht, neben der erfindungsgemäßen Wirkstoffkombination weiterhin eine Mischung aus 0,01 bis 2 Gew.-%, bevorzugt 0,05 bis 1,5 Gew.-% und insbesondere 0,1 bis 1 Gew.-% D-Panthenol und 0,01 bis 2 Gew.-%, bevorzugt 0,05 bis 1,5 Gew.-% und insbesondere 0,1 bis 1 Gew.-% Moringa-Extrakt.

Je nach gewünschter Behandlung kann das erfindungsgemäß verwendete Haarbehandlungsmittel als Shampoo, leave-in oder rinse-off Conditioner, Haarkur, Haarpflege-Sprühemulsion oder Schaumaerosol konfektionisert sein. Enthält das erfindungsgemäß verwendete Haarbehandlungsmittel zusätzlich noch mindestens ein kationisches Tensid, so handelt es sich üblicherweise um leave-on oder rinse-off-Conditioner, Haarkuren, Haarpflege-Sprühemulsionen oder Schaumaerosole.

Eine weitere Ausführungsform der vorliegenden Erfindung ist daher dadurch gegeben, dass das erfindungsgemäß verwendete Haarbehandlungsmittel als Shampoo, leave-in oder rinse-off Conditioner, Haarkur, Haarpflege-Sprühemulsion oder Schaumaerosol konfektioniert ist.

Gemäß einer weiteren bevorzugten Ausführungsform handelt es sich bei dem erfindungsgemäß verwendeten Haarbehandlungsmittel um ein Haarkonditioniermittel, das zusätzlich kationische Tenside enthält.

Die Erfindung betrifft daher ebenfalls die Verwendung solcher Haarbehandlungsmittel, die, bezogen auf ihr Gesamtgewicht, auf 0,1 bis 10 Gew.-%, bevorzugt 0,15 bis 7,5 Gew.-%, mehr bevorzugt 0,2 bis 6,5 Gew.-% und insbesondere 0,25 bis 5 Gew.-% kationischen Tensiden beruhen.

Dabei handelt es sich üblicherweise um leave-on oder rinse-off-Conditioner, Haarkuren, Haarpflege-Sprühemulsionen oder Schaumaerosole.

Kationische Tenside sind üblicherweise ausgewählt aus der Gruppe der quartären Ammoniumverbindungen, der Esterquats und der Amidoamine.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Behenyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 22 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Der Einsatz von kationischen Tensiden ist nicht auf erfindungsgemäß verwendete Haarkonditionierungsmittel beschränkt. Vielmehr können auch erfindungsgemäß verwendete Haarreinigungsmittel zur Steigerung der Hautverträglichkeit und Milde ebenfalls kationische Tenside enthalten.

In einer weiteren Ausführungsform der Erfindung enthalten die erfindungsgemäß verwendeten Haarbehandlungsmittel zur Steigerung der Hautmilde und zur Verringerung von Hautirritationen weiterhin mindestens ein nichtionisches Tensid in einer Menge von 0,1 bis 10 Gew.-%, bevorzugt von 0,15 bis 7,5 Gew.-%, mehr bevorzugt von 0,2 bis 6,5 Gew.-% und insbesondere von 0,25 bis 5 Gew.-%, bezogen auf das Gesamtgewicht des Haarbehandlungsmittels.

Solche nichtionischen Tenside sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe;
- mit einem Methyl- oder C₂-C₆-Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe;
- C₁₂-C₃₀-Fettsäuremono- und -diester von Anlagerungsprodukten von 1 bis 30 Mol Ethylenoxid an Glycerin;
- Polyglycerinester und alkoxylierte Polyglycerinester;
- Anlagerungsprodukte von 5 bis 60 Mol Ethylenoxid an ggf. gehärtetes Rizinusöl;
- alkoxylierte, bevorzugt propoxylierte und insbesondere ethoxylierte, Mono-, Di- und Triglyceride, wie beispielsweise Glycerinmonolaurat + 20 Ethylenoxid und Glycerinmonostearat + 20 Ethylenoxid;
- alkoxylierte Fettsäurealkylester der Formel RC(O)-(OCH₂CH₂)_{w}OR', in der RC(O)- für einen linearen oder verzweigten, gesättigten und/oder ungesättigten Acylrest mit 6 bis 22 Kohlenstoffatomen, R' für lineare oder verzweigte Alkylreste mit 1 bis 4 Kohlenstoffatomen und w für Zahlen von 1 bis 20 steht;
- Aminoxide;
- Sorbitanfettsäureester und Anlagerungeprodukte von Ethylenoxid an Sorbitanfettsäureester wie beispielsweise die Polysorbate, Sorbitanmonolaurat und Sorbitanmonolaurat + 20 Mol Ethylenoxid (EO);
- Zuckerfettsäureester und Anlagerungsprodukte von Ethylenoxid an Zuckerfettsäureester;
- Anlagerungsprodukte von Ethylenoxid an Fettsäurealkanolamide und Fettamine;
- Fettsäure-N-alkylglucamide;
- Alkylphenole und Alkylphenolalkoxylate mit 6 bis 21, insbesondere 6 bis 15 Kohlenstoffatomen in der Alkylkette und 0 bis 30 Ethylenoxid- und/oder Propylenoxid-Einheiten; Bevorzugte Vertreter dieser Klasse sind beispielsweise Nonylphenol + 4 EO, Nonylphenol + 9 EO, Octylphenol + 3 EO und Octylphenol + 8 EO;
- Alkylpolyglykoside entsprechend der allgemeinen Formel RO-(Z)ₓ, wobei R für Alkyl, Z für Zucker sowie x für die Anzahl der Zuckereinheiten steht.

Als nichtionische Tenside eignen sich insbesondere die Alkylenoxid-Anlagerungsprodukte an gesättigte lineare Fettalkohole und Fettsäuren sowie C₈-C₂₂-Alkylmono- und oligoglycoside und deren ethoxylierte Analoga. Insbesondere die nichtethoxylierten Verbindungen haben sich als besonders geeignet erwiesen.

Besonders bevorzugt sind solche Alkylpolyglykoside der Formel RO-(Z)ₓ, bei denen R aus C₈-C₁₀-Alkylgruppen, aus C₁₂-C₁₄-Alkylgruppen, im Wesentlichen aus C₈-C₁₆-Alkylgruppen, aus C₁₂-C₁₆-Alkylgruppen oder aus C₁₆-C₁₈-Alkylgruppen besteht. Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Eine weitere bevorzugte Ausführungsform der vorliegenden Erfindung besteht darin, dass das erfindungsgemäß verwendete anwendungsbereite Haarbehandlungsmittel einen pH-Wert zwischen 2,0 und 9,0, bevorzugt zwischen 2,5 und 7,0 besitzt. Insbesondere bevorzugt und besonders hautverträglich sind die Mittel Haarbehandlungsmittel in einem pH-Bereich von 3,0 bis 6,0. Bei den pH-Werten im Sinne der vorliegenden Erfindung handelt es sich um pH-Werte, die bei einer Temperatur von 22 °C gemessen wurden.

Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Neben den zwingenden Bestandteilen der erfindungsgemäß verwendeten Wirkstoffkombination können die erfindungsgemäß verwendeten Haarbehandlungsmittel in einer bevorzugten Ausführungsform noch weitere Haarpflegestoffe enthalten, die die erfindungsgemäße Wirkung noch weiter steigern bzw. unterstützen können.

Diese bevorzugten weiteren Wirkstoffe sind ausgewählt aus der Gruppe der natürlichen und synthetischen Ölkomponenten, der Antischuppenwirkstoffe, der kationischen Polymere, der UV-Filter und/oder der Silikone.

Erfindungsgemäß einsetzbar sind vorzugsweise kationische Polymere. Unter erfindungsgemäß geeigneten kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Ein geeignetes Homopolymer ist das, gewünschtenfalls vernetzte, Poly(methacroyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 und Salcare^{®} SC 96 im Handel erhältlich.

Copolymere mit den oben angeführten kationischen Monomereinheiten enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere sind im Handel unter der Bezeichnung Salcare^{®} SC 92 erhältlich.

Weitere bevorzugte kationische Polymere sind beispielsweise
- quaternisierte Cellulose-Derivate (wie unter den Bezeichnungen Celquat^{®} und Polymer JR^{®}, bevorzugt Celquat^{®} H 100, Celquat^{®} L 200 und Polymer JR^{®}400),
- hydrophob modifizierte Cellulosederivate (beispielsweise Handelsprodukt SoftCat^{®}),
- kationische Alkylpolyglycoside,
- kationiserter Honig (beispielsweise Handelsprodukt Honeyquat^{®} 50),
- kationische Guar-Derivate (beispielsweise Handelsprodukte Cosmedia^{®}Guar und Jaguar^{®}),
- Polysiloxane mit quaternären Gruppen (beispielsweise Handelsprodukte Q2-7224 (Dow Corning), Dow Corning^{®} 929 Emulsion, SM-2059 (General Electric), SLM-55067 (Wacker) sowie Abil^{®}-Quat 3270 und 3272 (Th. Goldschmidt; Quaternium-80),
- polymere Dimethyldiallylammoniumsalze und deren Copolymere (beispielsweise Handelsprodukte Merquat^{®}100 und Merquat^{®}550),
- Copolymere des Vinylpyrrolidinons mit quaternierten Derivaten des Dialkylaminoalkylacrylats und -methacrylats (beispielsweise Handelsprodukte Gafquat^{®}734 und Gafquat^{®}755),
- Vinylpyrrolidon-Vinylimidazoliummethochlorid-Copolymere (beispielsweise Handelsprodukte Luviquat^{®} FC 370, FC 550, FC 905 und HM 552),
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrölidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

In einer besonders bevorzugten Ausführungsform der Erfindung enthalten die erfindungsgemäß verwendeten Haarbehandlungsmittel weiterhin ein kationisches Polymer zur Steigerung der Abscheidung kosmetischer Haut- und Haarpflegewirkstoffe sowie zur Haut- und Haarkonditionierung. Bei diesem handelt es sich bevorzugt um ein kationisches Guar-Derivat und/oder Polyquaternium-7 (Merquat 550), Polyquaternium-6, Polyquaternium-10, Polyquaternium-67 (SoftCat^{®}-Polymere) und oder Salcare^{®} SC 95 bzw. Salcare^{®} SC 96. Das oder die kationische(n) Polymer(e) wird (werden) in den erfindungsgemäß verwendeten Mitteln Haarbehandlungsmitteln in einer Menge von 0,01 bis 10 Gew.-%, bevorzugt von 0,05 bis 5 Gew.-% und insbesondere von 0,1 bis 3 Gew.-%, bezogen auf das Gesamtgewicht des Haarbehandlungsmittels, eingesetzt.

Eine weitere erfindungsgemäß bevorzugte Komponente ist ein Öl, das unter natürlichen und synthetischen Ölkomponenten und/oder Fettstoffen ausgewählt sein kann. Insbesondere die erfindungsgemäßen Konditioniermittel enthalten mindestens eine Öl- und/oder Fettkomponente.

Als natürliche (pflanzliche) Öle werden üblicherweise Triglyceride und Mischungen von Triglyceriden eingesetzt. Bevorzugte natürliche Öle im Sinne der Erfindung sind Kokosnussöl, (süßes) Mandelöl, Walnussöl, Pfirsichkernöl, Aprikosenkernöl, Avocadoöl, Teebaumöl, Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Kakaobutter und Shea-Butter.

Als mineralische Öle kommen insbesondere Mineralöle, Paraffin- und Isoparaffinöle sowie synthetische Kohlenwasserstoffe zum Einsatz. Ein erfindungsgemäß einsetzbarer Kohlenwasserstoff ist beispielsweise das als Handelsprodukt erhältliche 1,3-Di-(2-ethylhexyl)-cyclohexan (Cetiol^{®} S).

Als Ölkomponente kann weiterhin ein Dialkylether dienen. Erfindungsgemäß einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether. Erfindungsgemäß besonders bevorzugt ist der Di-n-octylether, der im Handel unter der Bezeichnung Cetiol^{®} OE erhältlich ist.

Die erfindungsgemäß verwendeten Haarbehandlungsmittel enthalten die Ölkomponente(n) bevorzugt in einem Mengenbereich von 0,1 bis 7 Gew.-%, insbesondere von 0,25 bis 5 Gew.-%, bezogen auf das Gesamtgewicht des Haarbehandlungsmittels.

In einer weiteren bevorzugten Ausführungsform der Erfindung kann die Wirkung der erfindungsgemäß verwendeten Wirkstoffkombination durch weitere Fettstoffe noch weiter optimiert werden. Unter weiteren Fettstoffen sind Fettalkohole sowie natürliche und synthetische Wachse zu verstehen, welche sowohl in fester Form als auch flüssig in wässriger Dispersion vorliegen können.

Die Einsatzmenge beträgt dabei 0,1 bis 15 Gew.-%, bezogen auf das gesamte Haarbehandlungsmittel. In einer bevorzugten Ausführungsform beträgt die Menge 0,5 bis 10 Gew.-%, wobei ganz besonders vorteilhaft Mengen von 1 bis 5 Gew.-% sind.

Als Fettalkohole können eingesetzt werden gesättigte, ein- oder mehrfach ungesättigte, verzweigte oder unverzweigte Fettalkohole mit C₆-C₃₀-, bevorzugt C₁₀-C₂₂- und ganz besonders bevorzugt C₁₂-C₂₂-Alkylketten. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnussöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Weitere Fettstoffe sind beispielsweise
- Esteröle, worunter die Ester von C₆-C₃₀-Fettsäuren mit C₂-C₃₀-Fettalkoholen zu verstehen sind. Bevorzugt sind die Monoester der Fettsäuren mit C₂-C₂₄-Alkoholen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V);
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykoldi-pelargonat, Butandiol-di-isostearat, Neopentylglykoldicaprylat,

Antischuppenmittel tragen ebenfalls zur Verbesserung der erfindungsgemäß verwendeten Haarbehandlungsmittel bei, denn mit der Verringerung oder Inhibierung von Kopfschuppen gehen auch eine bessere Hautverträglichkeit und ein geringeres Irritationspotential der Kopfhaut einher. Erfindungsgemäß bevorzugt verwendete Haarbehandlungsmittel enthalten somit weiterhin mindestens einen kosmetisch akzeptablen Antischuppenwirkstoff. Dieser wird den erfindungsgemäß verwendeten Zusammensetzungen in einer Menge von 0,05 bis 5 Gew.-%, bevorzugt von 0,1 bis 3,0 Gew.-% und insbesondere von 0,3 bis 2,0 Gew.-% zugegeben (bezogen auf das gesamte Haarbehandlungsmittel) und ist ausgewählt aus Piroctone Olamine, Climbazol, Zink Pyrithion, Ketoconazole, Salicylsäure, Schwefel, Selensulfide, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten.Erfindungsgemäß bevorzugt sind Salicylsäure, Climbazol, Zink Pyrithion und Piroctone Olamine.

Weiterhin kann in einer bevorzugten Ausführungsform der Erfindung ein erfindungsgemäß verwendetes Haarbehandlungsmittel auch UV-Filter enthalten, da UV-Filter die Kopfhaut vor erhöhter Sonnenstrahleneinwirkung schützen. Des weiteren ist es bekannt, dass bestimmte Wirkstoffe in der Kombination mit erhöhter Sonnenlicht bzw. UV-Lichteinstrahlung Hautirritationen hervorrufen können, daher ist es erfindungsgemäß bevorzugt, wenn die Mittel weiterhin einen UV-Filter enthalten.

Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbar UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul^{®} M 40, Uvasorb^{®} MET, Neo Heliopan^{®}BB, Eusolex^{®}4360), 2-Phenylbenzimidazol-5-sulfonsäure und deren Kalium-, Natrium- und Triethanolaminsalze (Phenylbenzimidazole sulfonic acid; Parsol^{®}HS; Neo Heliopan^{®}Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol^{®}1789, Eusolex^{®}9020), α-(2-Oxoborn-3-yliden)-toluol-4-sulfonsäure und deren Salze, ethoxylierte 4-Aminobenzoesäure-ethylester (PEG-25 PABA; Uvinul^{®}P 25), 4-Dimethylaminobenzoesäure-2-ethylhexylester (Octyl Dimethyl PABA; Uvasorb^{®}DMO, Escalol^{®}507, Eusolex^{®}6007), Salicylsäure-2-ethylhexylester (Octyl Salicylat; Escalol^{®}587, Neo Heliopan^{®}OS, Uvinul^{®}O18), 4-Methoxyzimtsäure-isopentylester (Isoamyl p-Methoxycinnamate; Neo Heliopan^{®}E 1000), 4-Methoxyzimtsäure-2-ethylhexylester (Octyl Methoxycinnamate; Parsol^{®}MCX, Escalol^{®}557, Neo Heliopan^{®}AV), 2-Hydroxy-4-methoxybenzophenon-5-sulfonsäure und deren Natriumsalz (Benzophenone-4; Uvinul^{®}MS 40; Uvasorb^{®}S 5), 3-(4'-Methylbenzyliden)-D,L-Campher (4-Methylbenzylidene camphor; Parsol^{®}5000, Eusolex^{®}6300), 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-yliden-methyl]benzyl}-acrylamids, 2,4-Dihydroxybenzophenon (Benzophenone-1; Uvasorb^{®}20 H, Uvinul^{®}400), 1,1'-Diphenylacrylonitrilsäure-2-ethylhexyl-ester (Octocrylene; Eusolex^{®}OCR, Neo Heliopan^{®}Type 303, Uvinul^{®}N 539 SG), o-Aminobenzoesäure-menthylester (Menthyl Anthranilate; Neo Heliopan^{®}MA), 2,2',4,4'-Tetrahydroxybenzophenon (Benzophenone-2; Uvinul^{®}D-50), 2,2'-Dihydroxy-4,4'-dimethoxybenzophenon (Benzophenone-6), 2,2'-Dihydroxy-4,4'-dimethoxybenzo-phenon-5-natriumsulfonat und 2-Cyano-3,3-diphenylacrylsäure-2'-ethylhexylester. Bevorzugt sind 4-Amino-benzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Trianilino-(p-carbo-2'-ethylhexyl-1'-oxi)-1,3,5-triazin, 3-Imidazol-4-ylacrylsäure und deren Ethylester, Polymere des N-{(2 und 4)-[2-oxoborn-3-ylidenmethyl]benzyl}acrylamid. Erfindungsgemäß ganz besonders bevorzugt sind 2-Hydroxy-4-methoxy-benzophenon, 2-Phenyl-benzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyl-dimethylaminobenzamidopropyl-dimethylammoniumtosylat (Escalol^{®} HP 610).

Die UV-Filter sind in den erfindungsgemäß verwendeten Haarbehandlungsmitteln üblicherweise in Mengen von 0,1 bis 5 Gew.-%, bevorzugt von 0,4 bis 2,5 Gew.-%, bezogen auf das gesamte Mittel Haarbehandlungsmittel, enthalten.

Insbesondere bevorzugt sind erfindungsgemäß verwendete Haarbehandlungsmittel, die mindestens ein Silicon enthalten. Silicone bewirken auf dem Haar ausgezeichnete konditionierende Eigenschaften. Insbesondere bewirken sie eine bessere Kämmbarkeit der Haare in nassem und trockenem Zustand und wirken sich in vielen Fällen positiv auf den Haargriff und die Weichheit der Haare aus. Es ist daher erfindungsgemäß erstrebenswert, insbesondere bei Haarkonditioniermitteln mindestens eine Siliconkomponente hinzuzufügen. Diese(s) (ist) sind ausgewählt unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter substituierten oder unsubstituierten aminierten Gruppen; (per)fluorierten Gruppen; Thiolgruppen; Carboxylatgruppen; hydroxylierten Gruppen; alkoxylierten Gruppen; Acyloxyalkylgruppen; amphoteren Gruppen; Sulfinsäuregruppen; Hydroxyacylaminogruppen; Carboxygruppen; Sulfonsäuregruppen; und Sulfat- oder Thiosulfatgruppen;
(iii) linearen Polysiloxan(A)-Polyoxyalkylen(B)-Blockcopoylmeren;
(iv) gepfropften Siliconpolymeren mit nicht siliconhaltigem, organischen Grundgerüst;
(v) gepfropften Siliconpolymeren mit Polysiloxan-Grundgerüst;
(vi) oder deren Gemischen.

Die vorgenannten Silikonkomponenten werden den erfindungsgemäß verwendeten Haarbehandlungsmitteln - bezogen auf deren Gesamtgewicht - in einer Menge von 0,1 bis 10 Gew.-%, bevorzugt von 0,2 bis 7,5 Gew.-% und insbesondere von 0,3 bis 5 Gew.-% (bezogen auf den Aktivsubstanzgehalt des Silikons) hinzugefügt.

In einer weiteren bevorzugten Ausführungsform können die erfindungsgemäß verwendeten Haarbehandlungsmittel Emulgatoren enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W-Emulsionen und hydrophobe Emulgatoren bilden bevorzugt W/O-Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren

Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin;
- Ethylenoxid- und Polyglycerin-Anlagerungsprodukte an Methylglucosid-Fettsäureester, Fettsäurealkanolamide und Fettsäureglucamide;
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind;
- Gemische aus Alkyl-(oligo)-glucosiden und Fettalkoholen (z. B. Handelsprodukt Montanov^{®}68);
- Anlagerungsprodukte von 5 bis 60 Mol Ethylenoxid an ggf. gehärtetes Rizinusöl,
- Partialester von Polyolen mit 3-6 Kohlenstoffatomen mit gesättigten Fettsäuren mit 8 bis 22 C-Atomen,
- Sterine, insbesondere tierischem (Zoosterine, z. B. Cholesterin und Lanosterin) wie auch aus pflanzlichen Ursprungs (Phytosterine, z. B. Ergosterin, Stigmasterin und Sitosterin) sowie aus Pilzen und Hefen (Mykosterine);
- Phospholipide, vor allem Glucose-Phospolipide (z.B. als Lecithine bzw. Phosphatidylcholine aus z.B. Eidotter oder Pflanzensamen (z.B. Sojabohnen);
- Fettsäureester von Zuckern und Zuckeralkoholen, wie Sorbit;
- Polyglycerine und Polyglycerinderivate.

Die erfindungsgemäß verwendeten Haarbehandlungsmittel enthalten die Emulgatoren bevorzugt in Mengen von 0,1 - 25 Gew.-%, insbesondere 0,5 - 15 Gew.-%, bezogen auf das gesamte Haarbehandlungsmittel.

Bevorzugt können die erfindungsgemäß verwendeten Zusammensetzungen mindestens einen nichtionogenen Emulgator mit einem HLB-Wert von 8 bis 18 enthalten. Nichtionogene Emulgatoren mit einem HLB-Wert von 10 bis15 können erfindungsgemäß besonders bevorzugt sein.

Ferner können die erfindungsgemäß verwendeten Haarbehandlungsmittel weitere Wirk-, Hilfs- und Zusatzstoffe enthalten, insbesondere
- nichtionische Polymere wie beispielsweise Vinylpyrrolidinon/Vinylacrylat-Copolymere, Polyvinylpyrrolidinon und Vinylpyrrolidinon/Vinylacetat-Copolymere und Polysiloxane,
- zwitterionische und amphotere Polymere wie beispielsweise Acrylamidopropyl-tri-methylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert-Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- Parfümöle, Dimethylisosorbid und Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe und Pigmente zum Anfärben des Mittels,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Treibmittel wie Propan-Butan-Gemische, N₂O, Dimethylether, CO₂ und Luft, sowie
- Antioxidantien.

Die Auswahl dieser weiteren Stoffe wird der Fachmann gemäß der gewünschten Eigenschaften der erfindungsgemäß verwendeten Haarbehandlungsmittel treffen.

Die folgenden Beispiele erläutern die Erfindung, ohne sie jedoch darauf einzuschränken:
Alle Angaben sind - sofern nicht anders angegeben - in Gewichtsprozent.

### Beispiele:

### Haarpflegeshampoo 1:

| | |
|---|---|
| Ethoxyliertes Fettalkoholsulfat (C₁₂-C₁₄, 2 EO; 70% AS) | 15,0 |
| Disodium Cocoamphodiacetate | 7,0 |
| Euperlan^{®}PK 3000 AM¹ | 2,5 |
| Coconut Glycerides + 7,3 EO | 0,5 |
| Cutina HR^{®2} | 0,1 |
| D-Panthenol | 0,5 |
| 4-Acetyl-1-methylpyridinium-p-toluolsulfonat³ | 0,5 |
| Zitronensäure | 0,5 |
| Salicylsäure | 0,2 |
| Glycerin | 0,15 |
| Sodium Benzoate | 0,4 |
| NaCl | 1,0 |
| Parfum | qs |
| Wasser | ad 100 |

### Haarpflegeshampoo 2:

| | |
|---|---|
| Ethoxyliertes Fettalkoholsulfat (C₁₂-C₁₄, 2 EO; 70% AS) | 15,0 |
| Cocamidopropylbetain | 5,0 |
| Plantacare^{®4} 818 UP | 4,0 |
| Coconut Glycerides + 7,3 EO | 0,5 |
| D-Panthenol | 0,5 |
| Glycin | 0,2 |
| 4-Acetyl-1-methylpyridinium-p-toluolsulfonat | 0,3 |
| Zitronensäure | 0,5 |
| Sodium Benzoate | 0,4 |
| Glycerin | 0,1 |
| NaCl | 1,0 |
| Parfum | qs |
| Wasser | ad 100 |

### Haarpflegeshampoo 3:

| | |
|---|---|
| Ethoxyliertes Fettalkoholsulfat (C₁₂-C₁₄, 2 EO; 70% AS) | 15,0 |
| Cocamidopropylbetain | 2,0 |
| Disodium Cocoamphodiacetate | 3,0 |
| Plantacare^{®4} 818 UP | 4,0 |
| Coconut Glycerides + 7,3 EO | 0,5 |
| ProSina^{®5} | 0,5 |
| 4-Acetyl-1-methylpyridinium-p-toluolsulfonat | 0,75 |
| Zitronensäure | 0,5 |
| Sodium Benzoate | 0,4 |
| Mannitol | 0,1 |
| NaCl | 1,0 |
| Parfum | qs |
| Wasser | ad 100 |

### Haarpflegeshampoo 4:

| | |
|---|---|
| Ethoxyliertes Fettalkoholsulfat (C₁₂-C₁₄, 2 EO; 70% AS) | 15,0 |
| Cocamidopropylbetain | 5,0 |
| Plantacare^{®4} 818 UP | 4,0 |
| Coconut Glycerides + 7,3 EO | 0,5 |
| D-Panthenol | 0,5 |
| Ectoin | 0,2 |
| 4-Acetyl-1-methylpyridinium-p-toluolsulfonat | 0,5 |
| Zitronensäure | 0,5 |
| Sodium Benzoate | 0,4 |
| Sorbitol | 0,1 |
| NaCl | 1,0 |
| Parfum | qs |
| Wasser | ad 100 |

Es wurden die folgenden Handelsprodukte eingesetzt:
1 INCI-Bezeichnung: Aqua, Glycol Distearate, Glycerin, Laureth-4, Cocamidopropyl Betaine, Formic Acid (Solids: 41-45%); Cognis
2 INCI-Bezeichnung: Hydrogenated Castor Oil; Cognis
3 Zur Herstellung von 4-Acetyl-1-methylpyridinium-p-toluolsulfonat, vgl. Offenlegungsschrift DE 102007047685 A1
4 INCI-Bezeichnung: Coco-Glucoside (AS 51-53%); Cognis
5 INCI-Bezeichnung: Aqua, Hydrolyzed Keratin; Croda

## Patentansprüche

1. Verwendung eines kosmetischen Haarbehandlungsmittels, das frei ist von Wasserstoffperoxid und/oder dessen Anlagerungsverbindungen an organische und anorganische Verbindungen und das in einem kosmetischen Träger
a) mindestens ein oberflächenaktives Mittel, ausgewählt aus der Gruppe der anionischen, amphoteren und/oder zwitterionischen Tenside, und
b) mindestens ein Acetylpyridiniumderivat der Formel (I), 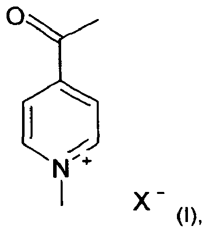 worin
R1 für eine C₁-C₆-Alkylgruppe, eine C₂-C₆-Alkenylgruppe, eine C₂-C₆-Hydroxyalkylgruppe, eine C₁-C₆-Alkoxy-C₂-C₆-alkylgruppe, eine Carboxy-C₂-C₆-alkylgruppe, eine Aryl-C₁-C₆-alkylgruppe, eine Heteroaryl-C₁-C₆-alkylgruppe, eine Arylgruppe oder eine Heteroarylgruppe und
X- für ein physiologisch verträgliches Anion stehen,
enthält,
zur Reduktion von Haarschädigungen im Inneren der Haare und zur Steigerung der Elastizität der Haare.

2. Verwendung nach Anspruch 1, **dadurch gekennzeichnet, dass** der Rest R1 für eine C₁-C₆-Alkylgruppe, eine C₂-C₆-Alkenylgruppe oder eine C₂-C₆-Hydroxyalkylgruppe steht.

3. Verwendung nach einem der Ansprüche 1 oder 2, **dadurch gekennzeichnet, dass** das Acetylpyridiniumderivat gemäß Formel (I) ausgewählt ist aus der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumbromid, 4-Acetyl-1-methylpyridiniumchlorid, 4-Acetyl-1-methylpyridiniumhydrogensulfat, 4-Acetyl-1-methylpyridiniumacetat, 4-Acetyl-1-allylpyridinium-p-toluolsulfonat, 4-Acetyl-1-allylpyridinium-benzolsulfonat, 4-Acetyl-1-allylpyridiniumbromid, 4-Acetyl-1-allylpyridiniumchlorid, 4-Acetyl-1-allylpyridiniumhydrogensulfat und/oder 4-Acetyl-1-allylpyridiniumacetat.

4. Verwendung nach einem der Ansprüche 1 bis 3, **dadurch gekennzeichnet, dass** das Acetylpyridiniumderivat gemäß Formel (I) ausgewählt ist aus 4-Acetyl-1-methylpyridinium-p-toluolsulfonat.

5. Verwendung nach einem der Ansprüche 1 bis 4, **dadurch gekennzeichnet, dass** das Haarbehandlungsmittel das Acetylpyridiniumderivat gemäß Formel (I) zu 0,05 bis 10 Gew.-%, bevorzugt 0,1 bis 7,5 Gew.-%, bevorzugt 0,2 bis 6,5 Gew.-% und insbesondere 0,5 bis 5 Gew.-%, jeweils bezogen auf das Gesamtgewicht des anwendungsbereiten Haarbehandlungsmittels, enthält.

6. Verwendung nach einem der Ansprüche 1 bis 5, **dadurch gekennzeichnet, dass** das Haarbehandlungsmittel als oberflächenaktives Mittel mindestens ein anionisches Tensid enthält.

7. Verwendung nach einem der Ansprüche 1 bis 6, **dadurch gekennzeichnet, dass** das Haarbehandlungsmittel die oberflächenaktiven Mittel, ausgewählt aus der Gruppe der anionischen, amphoteren und/oder zwitterionischen Tenside, in einer Gesamtmenge von 0,1 bis 50 Gew.-%, bevorzugt 0,5 bis 25 Gew.-%, mehr bevorzugt 1 bis 20 Gew.-% und insbesondere 2 bis 15 Gew.-%, jeweils bezogen auf das Gesamtgewicht des anwendungsbereiten Haarbehandlungsmittels, enthält.

8. Verwendung nach einem der Ansprüche 1 bis 7, **dadurch gekennzeichnet, dass** das Haarbehandlungsmittel weiterhin mindestens einen Pflegestoff aus der Gruppe der Proteinhydrolysate und/oder der Vitamine und/oder der Aminosäuren und/oder der Pflanzenextrakte und/oder Ectoin enthält.

9. Verwendung nach Anspruch 8, **dadurch gekennzeichnet, dass** das Haarbehandlungsmittel als zusätzliche Pflegekomponente D-Panthenol, Keratinhydrolysat, Glycin, Serin, Arginin, Moringa Oleifera-Extrakt und/oder Ectoin, enthält.

10. Verwendung nach einem der Ansprüche 1 bis 9, **dadurch gekennzeichnet, dass** das Haarbehandlungsmittel als Shampoo, leave-in oder rinse-off Conditioner, Haarkur, Haarpflege-Sprühemulsion oder Schaumaerosol konfektioniert ist.

## Claims

1. Use of a cosmetic hair treatment product which is free of hydrogen peroxide and/or its addition compounds to organic and inorganic compounds, and which contains, in a cosmetic carrier,
a) at least one surface-active agent selected from the group of anionic, amphoteric, and/or zwitterionic surfactants, and
b) at least one acetylpyridinium derivative of formula (I), 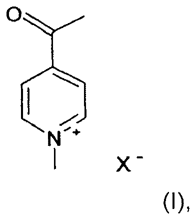 where
R1 stands for a C₁-C₆ alkyl group, a C₂-C₆ alkenyl group, a C₂-C₆ hydroxyalkyl group, a C₁-C₆ alkoxy-C₂-C₆ alkyl group, a carboxy-C₂-C₆ alkyl group, an aryl-C₁-C₆ alkyl group, a heteroaryl-C₁-C₆ alkyl group, an aryl group, or a heteroaryl group, and
X- stands for a physiologically acceptable anion,
for reducing internal damage to the hair and for increasing the elasticity of the hair.

2. Use according to Claim 1, **characterized in that** the radical R1 stands for a C₁-C₆ alkyl group, a C₂-C₆ alkenyl group, or a C₂-C₆ hydroxyalkyl group.

3. Use according to one of Claims 1 and 2, **characterized in that** the acetylpyridinium derivative according to formula (I) is selected from the group comprising 4-acetyl-1-methylpyridinium *p*-toluene sulfonate, 4-acetyl-1-methylpyridinium benzene sulfonate, 4-acetyl-1-methylpyridinium bromide, 4-acetyl-1-methylpyridinium chloride, 4-acetyl-1-methylpyridinium hydrogen sulfate, 4-acetyl-1-methylpyridinium acetate, 4-acetyl-1-allylpyridinium *p*-toluene sulfonate, 4-acetyl-1-allylpyridinium benzene sulfonate, 4-acetyl-1-allylpyridinium bromide, 4-acetyl-1-allylpyridinium chloride, 4-acetyl-1-allylpyridinium hydrogen sulfate, and/or 4-acetyl-1-allylpyridinium acetate.

4. Use according to one of Claims 1 to 3, **characterized in that** the acetylpyridinium derivative according to formula (I) is selected from 4-acetyl-1-methylpyridinium p-toluene sulfonate.

5. Use according to one of Claims 1 to 4, **characterized in that** the hair treatment product contains the acetylpyridinium derivative according to formula (I) in a quantity of 0.05 to 10% by weight, preferably 0.1 to 7.5% by weight, preferably 0.2 to 6.5% by weight, and in particular 0.5 to 5% by weight, in each case based on the total weight of the ready-to-use hair treatment product.

6. Use according to one of Claims 1 to 5, **characterized in that** the hair treatment product contains at least one anionic surfactant as surface-active agent.

7. Use according to one of Claims 1 to 6, **characterized in that** the hair treatment product contains the surface-active agents, selected from the group of anionic, amphoteric, and/or zwitterionic surfactants, in a total quantity of 0.1 to 50% by weight, preferably 0.5 to 25% by weight, more preferably 1 to 20% by weight, and in particular 2 to 15% by weight, in each case based on the total weight of the ready-to-use hair treatment product.

8. Use according to one of Claims 1 to 7, **characterized in that** the hair treatment product further contains at least one care substance from the group of protein hydrolysates and/or vitamins and/or amino acids and/or plant extracts and/or ectoine.

9. Use according to Claim 8, **characterized in that** the hair treatment product contains D-panthenol, keratin hydrolysate, glycine, serine, arginine, moringa oleifera extract, and/or ectoine as additional care component.

10. Use according to one of Claims 1 to 9, **characterized in that** the hair treatment product is in the form of a shampoo, leave-in or rinse-off conditioner, hair mask, hair care spray emulsion, or aerosol foam.

## Revendications

1. Utilisation d'un agent de traitement capillaire cosmétique, qui est dépourvu de peroxyde d'hydrogène et/ou de ses composés d'addition à des composés organiques et minéraux et qui contient dans un support cosmétique
a) au moins un agent tensioactif, choisi dans le groupe des tensioactifs anioniques, amphotères et/ou zwitterioniques et
b) au moins un dérivé de l'acétylpyridinium de la formule (I), 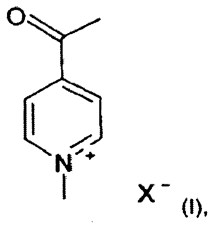 où
R1 représente un groupe alkyle en C₁-C₆, un groupe alcényle en C₂-C₆, un groupe hydroxyalkyle en C₂-C₆, un groupe alcoxy en C₁-C₆-alkyle en C₂-C₆, un groupe carboxy-alkyle en C₂-C₆, un groupe aryle-alkyle en C₁-C₆, un groupe hétéroaryl-alkyle en C₁-C₆, un groupe aryle ou un groupe hétéroaryle et
X représente un anion physiologiquement acceptable,
dans la réduction de dommages à l'intérieur des cheveux et l'amélioration de l'élasticité des cheveux.

2. Utilisation selon la revendication 1, **caractérisée en ce que** le radical R1 représente un groupe alkyle en C₁-C₆, un groupe alcényle en C₂-C₆ ou un groupe hydroxyalkyle en C₂-C₆.

3. Utilisation selon l'une quelconque des revendications 1 ou 2, **caractérisée en ce que** le dérivé de l'acétylpyridinium selon la formule (I) est choisi dans le groupe qui est formé par le 4-acétyl-1-méthylpyridinium-p-toluène sulfonate, le 4-acétyl-1-méthylpyridinium-benzène sulfonate, le bromure de 4-acétyl-1-méthylpyridinium, le chlorure de 4-acétyl-1-méthylpyridinium, l'hydrogénosulfate de 4-acétyl-1-méthylpyridinium, l'acétate de 4-acétyl-1-méthylpyridinium, le 4-acétyl-1-allylpyridinium-p-toluène sulfonate, le 4-acétyl-1-allylpyridinium-benzène sulfonate, le bromure de 4-acétyl-1-allylpyridinium, le chlorure de 4-acétyl-allylpyridinium, l'hydrogènosulfate de 4-acétyl-1-allylpyridinium et/ou l'acétate de 4-acétyl-1-allylpyridinium.

4. Utilisation selon l'une des revendications 1 à 3, **caractérisée en ce que** le dérivé de l'acétylpyridinium selon la formule (I) choisi est le 4-acétyl-1-méthylpyridinium-p-toluène sulfonate.

5. Utilisation selon l'une des revendications 1 à 4, **caractérisée en ce que** l'agent de traitement capillaire contient le dérivé de l'acétylpyridinium selon la formule (I) dans une quantité de 0,05 à 10 % en poids, de préférence de 0,1 à 7,5 % en poids, de préférence de 0,2 à 6,5 % en poids et en particulier de 0,5 à 5 % en poids, à chaque fois rapportée au poids total de l'agent de traitement capillaire prêt à l'emploi.

6. Utilisation selon l'une des revendications 1 à 5, **caractérisée en ce que** la composition de traitement capillaire comprend comme agent tensioactif au moins un tensioactif anionique.

7. Utilisation selon l'une des revendications 1 à 6, **caractérisée en ce que** l'agent de traitement capillaire contient l'agent tensioactif choisi dans le groupe des tensioactifs anioniques, amphotères et/ou zwitterioniques, dans une quantité totale de 0,1 à 50 % en poids, de préférence de 0,5 à 25 % en poids, plus préférablement de 1 à 20 % en poids et en particulier de 2 à 15 % en poids, à chaque fois rapportée au poids total de l'agent de traitement capillaire prêt à l'emploi.

8. Utilisation selon l'une des revendications 1 à 7, **caractérisée en ce que** la composition de traitement capillaire contient en outre au moins une substance de soin choisie dans le groupe des hydrolysats de protéines et/ou des vitamines et/ou des acides aminés et/ou des extraits végétaux et/ou de l'ectoïne.

9. Utilisation selon la revendication 8, **caractérisée en ce que** l'agent de traitement capillaire contient comme composant de soin supplémentaire le D-panthénol, l'hydrolysat de kératine, la glycine, la sérine, l'arginine, l'extrait de Moringa Oleifera et/ou l'ectoïne.

10. Utilisation selon l'une des revendications 1 à 9, **caractérisée en ce que** la composition de traitement capillaire est formulée sous la forme d'un shampooing, d'un flacon avec ou sans rinçage, d'un traitement capillaire, d'une émulsion de pulvérisation de soin capillaire ou d'une mousse aérosol.
